# EUROPÄISCHE PATENTSCHRIFT

(11) **EP 0 840 802 B1**
(45) Veröffentlichungstag und Bekanntmachung des Hinweises auf die Patenterteilung: **23.01.2002**
(21) Anmeldenummer: 96928329.0
(22) Anmeldetag: 25.07.1996
(51) Int. Cl.: C12Q 1/68, C07H 21/00, G01N 33/543

(54) **VERFAHREN ZUR SEQUENZIERUNG DURCH OLIGOMERHYBRIDISIERUNG**
METHOD OF SEQUENCING BY OLIGOMER HYBRIDISATION
PROCEDE DE SEQUENCAGE PAR HYDRIDATION D'OLIGOMERES

(30) Priorität: 25.07.1995 DE 19527155
(43) Veröffentlichungstag der Anmeldung: 13.05.1998
(73) Patentinhaber: Deutsches Krebsforschungszentrum Stiftung des öffentlichen Rechts, 69120 Heidelberg (DE)
(72) Erfinder: HOHEISEL, Jörg, D-69168 Wiesloch (DE)
(74) Vertreter: Schüssler, Andrea, Dr.
(86) Internationale Anmeldenummer: DE9601386
(87) Internationale Veröffentlichungsnummer: WO9705276

(56) Entgegenhaltungen:
- EP-A- 0 260 032
- WO-A-90/04652
- US-A- 5 403 711
- NUCLEIC ACID RESEARCH, Bd. 22, Nr. 8, 1994, Seiten 1444-1449, XP002023768 SANGHANI AND LAVERY: "Theoretical studies of DNA-RNA hybrid conformations"
- BIOCHEMISTRY, Bd. 32, 1993, Seiten 4207-4215, XP002023769 SALAZAR ET AL.: "The DNA strand in DNA/RNA hybrid duplexes is neither B-form nor A-form in solution"
- STRYER: "Biochemistry" 1988 , FREEMAN AND COMPANY , NY XP002023771 156460 siehe Seite 649 - Seite 653
- ANALYTICAL BIOCHEMISTRY, Bd. 224, Nr. 1, 1.Januar 1995, Seiten 110-116, XP000486745 MATSON R S ET AL: "BIOPOLYMER SYNTHESIS ON POLYPROPYLENE SUPPORTS: OLIGONUCLEOTIDE ARRAYS"
- NUCLEIC ACIDS RESEARCH, Bd. 22, Nr. 24, 11.Dezember 1994, Seiten 5456-5465, XP002006248 GUO Z ET AL: "DIRECT FLUORESCENCE ANALYSIS OF GENETIC POLYMORPHISMS BY HYBRIDIZATION WITH OLIGONUCLEOTIDE ARRAYS ON GLASS SUPPORTS"
- NUCLEIC ACID RESEARCH, Bd. 24, Nr. 3, 1.Februar 1996, Seiten 430-432, XP002023770 HOHEISEL: "Sequence-independent and linear variation of oligonucleotide DNA binding stabilities."

## Beschreibung

Die vorliegende Erfindung betrifft ein neues Verfahren zur Sequenzierung durch Oligomerhybridisierung.

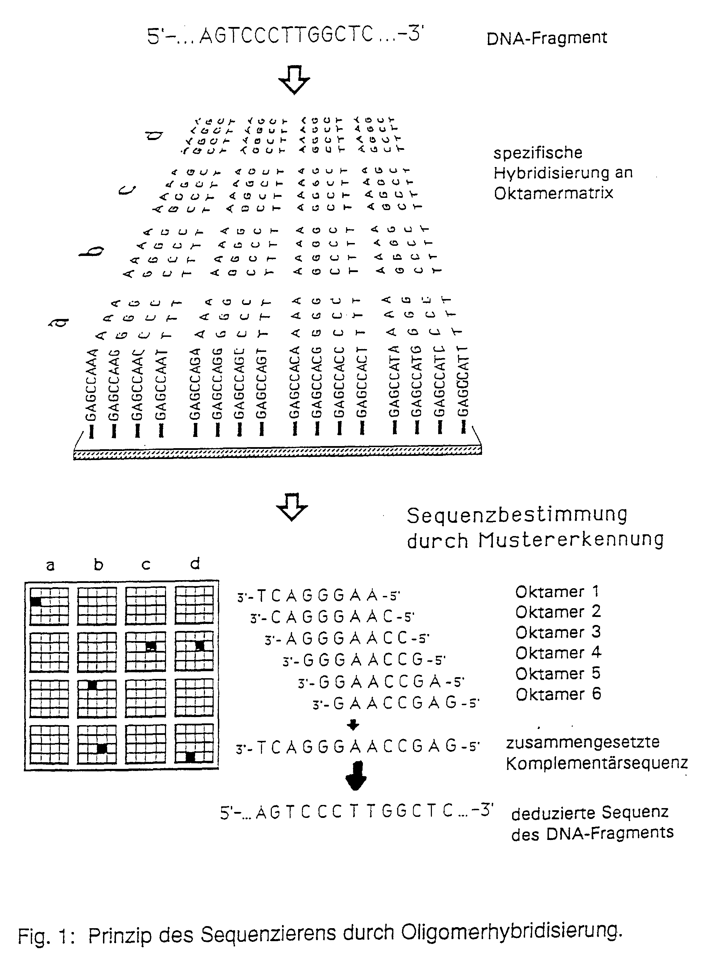

Vor einigen Jahren hat sich neben der lang bekannten DNA-Sequenzierung durch Auftragen auf Gele, ein weiteres Verfahren entwickelt, nämlich die Sequenzierung durch Oligomerhybridisierung (vgl. z.B. Drmanac et al., Genomics 4, (1989), S. 114-128 oder Bains et al., J. Theor. Biol. 135, (1988), S. 303-307). Für dieses Sequenzierungsverfahren wird ein vollständiger Satz kurzer Oligonukleotide bzw. Oligomere (z.B. alle denkbaren 65.536 Oktamere) in einem geordneten Raster auf ein Trägermaterial gebunden, so daß die Position jedes einzelnen Oligonukleotids bekannt ist. Auf solch einer Hybridisierungsmatrix bzw. einem "Oligomer-Chip" wird ein DNA-Fragment, dessen Sequenz man ermitteln will, hybridisiert, und zwar unter Bedingungen, die nur eine spezifische Doppelstrangbildung erlauben. Dadurch bindet das DNA-Fragment nur an die Oligomere, deren komplementäre Sequenz exakt einem Teil (z.B. einem Oktamer bei Verwendung eines "Oktamer-Chips") seiner eigenen Sequenz entspricht. Durch Detektion der Bindungspositionen des hybridisierten DNA-Fragments werden damit alle im Fragment vorhandenen Oligomersequenzen bestimmt. Aufgrund der Überlappung benachbarter Oligomersequenzen kann durch geeignete mathematische Algorithmen die fortlaufende Sequenz des DNA-Fragments bestimmt werden. Beispielhaft ist dieses Prinzip in Fig. 1 für ein 13 Basen langes DNA-Fragment, dessen Sequenz durch Oktamerhybridisierungs-Sequenzierung bestimmt werden soll, gezeigt.

Ein Hauptproblem bei der praktischen Anwendung dieser Methode liegt darin, daß die Oligomere in Abhängigkeit ihrer Sequenz eine unterschiedliche Bindungsstärke (Dissoziationstemperatur) besitzen, aber unter einem einzigen Satz experimenteller Hybridisierungstemperaturen spezifisch und in voller Länge an das zu bestimmende DNA-Fragment binden müssen. Bei Verwendung hoher Stringenz binden jedoch schwach bindende Oligomere nicht an das DNA-Fragment, obwohl ihre komplementäre Sequenz dort enthalten ist (falsch Negative). Bei geringer Stringenz dagegen binden auch Teilbereiche von Oligomeren (z.B. nur sechs oder sieben Nukleotide eines Oktamers) mit hoher Bindungsstärke, wodurch falsch Positive entstehen. Durch die Breite der Stabilitätsunterschiede treten unter den meisten Bedingungen beide Artefakte auf. Zur Nutzung dieser Sequenzierungstechnik ist es deshalb notwendig, die Bindungsstärke aller Oligomere, unabhängig von ihrer Sequenz, auf ein Niveau zu bringen. Dies geschieht bisher dadurch, daß die Oligomerkonzentration variiert wird (Khrapko et al., (1991) DNA Sequence 1, S. 375-388). Dies hat jedoch den Nachteil, daß große technische Schwierigkeiten bei der Herstellung der Hybridisierungsmatrix auftauchen, da für jedes Oligomer zusätzlich eine spezifische Konzentration eingestellt werden muß. Ein zweiter Ansatz, um die Bindungsstärke aller Oligomere auf ein Niveau zu bringen, ist die Einführung von Basenderivaten, die zu einer Änderung der Stabilität der jeweiligen Basenpaarung führen. Daran ist nachteilig, daß viele unterschiedliche Derivate verwendet werden müssen und durch die Einführung der Derivate die Spezifität der Basenpaarung geändert wird. Eine dritte Lösung zur Modulation der Bindungsstärke der Oligomere sieht die Hybridisierung in Tetraalkylammoniumsalzen vor, durch die die Stabilitätsunterschiede zwischen G:C und A:T Basenpaare verringert werden (Wood et al., (1985), Proc. Natl. Acad. Sci. 82, 1585-1588). Dieser Effekt ist jedoch für kurze Oligomere unzureichend. Außerdem behindert die Verwendung der hochmolaren Salzlösungen die Hybridisierung.

WO-A-9 004 652 betrifft die Sequenzierung von DNA durch Oligomerhybridisierung, wobei die Unterschiede in den Bindungsenergien durch Variation der Hybridisierungsbedingungen und/oder Verwendung von Nukleotidanaloga minimiert werden.

EP-A-0 260 032 betrifft Verbindungen für die Spaltung an einer spezifischen Position von RNA sowie Oligomere, die für die Bildung dieser Verbindungen verwendet werden.

US-A-5 403 711 betrifft Nukleinsäurehydridisierungs- und Amplifikationsmethoden für die Detektion spezieller Sequenzen.

Aufgabe der vorliegenden Anmeldung war es deshalb, in einem Verfahren zur Sequenzierung durch Oligomerhybridisierung, die Bindungsstärke aller Oligomere auf ein Niveau zu bringen, wobei die oben beschriebenen Nachteile vermieden werden sollen.

Gelöst wird diese Aufgabe durch ein Verfahren gemäß Anspruch 1. Vorteilhafte Ausgestaltungen ergeben sich aus den Unteransprüchen.

Von den Erfindern wurde herausgefunden, daß durch Verwendung von DNA/RNA-Hybridoligomeren, ggfs. in Kombination mit DNA- und/oder RNA-Oligomeren, auf der Hybridisierungsmatrix bei der Sequenzierung von unbekannten DNA-Fragmenten, die Bindungsstärke (Doppelstrangstabilität) durch die Einflußnahme auf die Zuckerfaltung variiert werden kann, und zwar ohne eine Beeinflussung der Spezifität der Basenpaarung. Gleichzeitig ist die Variation der Bindungsstärke durch Einsatz der DNA/RNA-Hybride unabhängig von der Basensequenz des Oligomers.

Die Synthese der DNA/RNA-Hybridoligomere erfolgt mit den üblichen bekannten Methoden, die zur Synthese reiner DNA-Oligonukleotide entwickelt wurden, wobei die notwendige Zahl an Nukleotiden nur verdoppelt werden muß (DNA- bzw. RNA-Nukleotid von A, G, C und T). Die Synthese erfolgt vorzugsweise in den auf dem Markt bekannten kommerziell erhältlichen Geräten oder leicht adaptierten Geräten. Die Aufbringung der einzelnen Oligomere zur Herstellung der Hybridisierungsmatrixerfolgt vorzugweise über individuellen Transfer gewisser Mengen unter Verwendung eines kommerziellen Roboters an eine bestimmte Position eines Trägers. Materialien für den Träger sind dem Fachmann geläufig, wobei vorzugsweise Glas, Polypropylen oder Silicium eingesetzt werden. Die Bindung der Oligomere an die Trägeroberfläche erfolgt vorzugsweise über dem Fachmann bekannte kommerziell erhältliche Linker, welche gegebenenfalls noch chemisch modifiziert worden sind.

Es besteht aber auch die Möglichkeit der gleichzeitigen Synthese aller Oligomere auf dem Träger. Dafür werden die Synthesebausteine in vier parallelen Kanälen (je einer für die Bausteine A, G, C, T mit entweder Desoxyribose oder Ribose-Zuckeranteil) über den Träger geleitet. Im zweiten Syntheseschritt liegen diese Kanäle senkrecht zu ihrer Ausrichtung im ersten Schritt. Im dritten und vierten Schritt werden für jeden einzelnen Kanal der Syntheseschritte 1 und 2 vier Kanäle für die Bausteine A, G, C, T verwendet, wodurch insgesamt 16 Kanäle, erst in der einen Dimension, dann in der zweiten Dimension angewendet werden. Für jedes weitere Paar an Syntheseschritten wird die Zahl an Kanälen jeweils vervierfacht. Allgemein ist dieses Verfahren in Southern et al., (1992), Genomics 13, S. 1008-1017 beschrieben, worauf hier Bezug genommen wird.

Ein weiteres Verfahren zur gleichzeitigen Synthese aller Oligomere auf dem Träger ist von Pease et al., ( 1994), Proc. Natl. Acad. Sci 91, S. 5022-5026 beschrieben worden. Dort werden unter Verwendung photoaktivierbarer Bausteine und photolitographischer Techniken die Oligonukleotide auf dem Träger synthetisiert. Der Träger wird mit einer Maske abgedeckt und mit Licht bestrahlt. Nur an den Stellen, an denen die Maske Löcher hat, wird die Oberfläche durch den Lichteinfall aktiviert. Nur an dieser aktivierten Position kann im nachfolgenden Syntheseschritt ein DNA- oder RNA-Nukleotid eingebaut werden. Für den folgenden Syntheseschritt wird eine neue Maske mit Löchern an einer anderen Position aufgelegt, die Oberfläche an dieser neuen Position mit Licht aktiviert und ein neues DNA- oder RNA-Nukleotid eingebaut.

In der Praxis hat es sich bewährt, für die Sequenzierung von DNA-Fragmenten bis zu 200 Basen eine Hybridisierungsmatrix, auf die Oktamere aufgebracht worden sind ("Oktamer-Chip") zu verwenden, während DNA von 200 Basen bis zu einer Kilobase Länge besser unter Verwendung eines "Decamer-Chips" sequenziert wird. Die Auswahl der geeigneten Größe der Oligomere obliegt dem Können eines Fachmanns und umfaßt vorzugsweise eine Tetramer- bis Dodecamer-Hybridisierungsmatrix.

Grundlage der vorliegenden Erfindung waren folgende Experimente an zwei verschiedenen Dodekamersequenzen, wobei Großbuchstaben DNA-Nukleotide und Kleinbuchstaben RNA-Nukleotide bedeuten. Es wurden folgende Oligomere hergestellt:

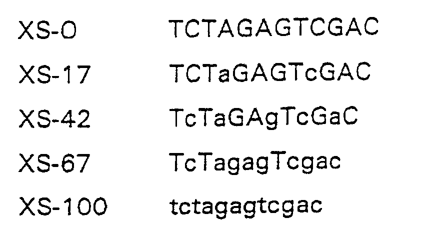

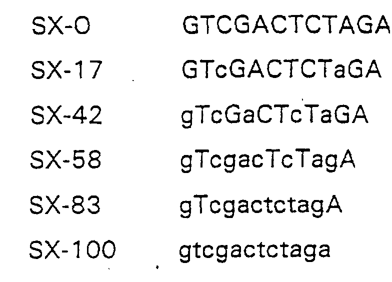

Es wurden jeweils gleiche Mengen radioaktiv markiertes Oligomer mit Einzelstrang-DNA gemischt, die die komplementäre Sequenz enthält. Unspezifische Bindung fand nicht statt. Das Einzelstrang-DNA-Oligomer-Gemisch wurde auf Dünnschichtplatten aufgebracht. Dünnschicht-Chromatographie fand unter Hybridisierungsbedingungen statt, wobei quer zur Laufrichtung ein Temperaturgradient angelegt war. Unter diesen Bedingungen läuft freies Oligomer mit der Pufferlauffront, während gebundenes Oligomer am Ausgangspunkt zurückbleibt. Eine Messung der Radioaktivität am Ausgangspunkt erlaubt eine Quantifizierung der Menge gebundenen Oligomers. Detailliert ist diese Meßmethode von Hoheisel et al., J. Biol. Chem. 165, S. 16656-16660 (1990) beschrieben worden.

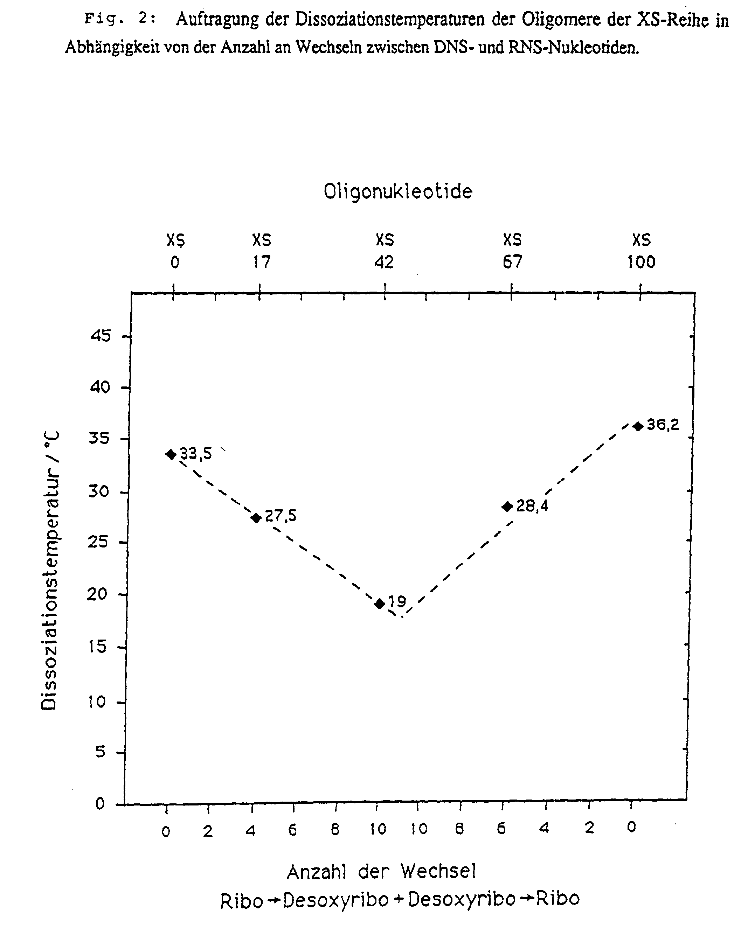

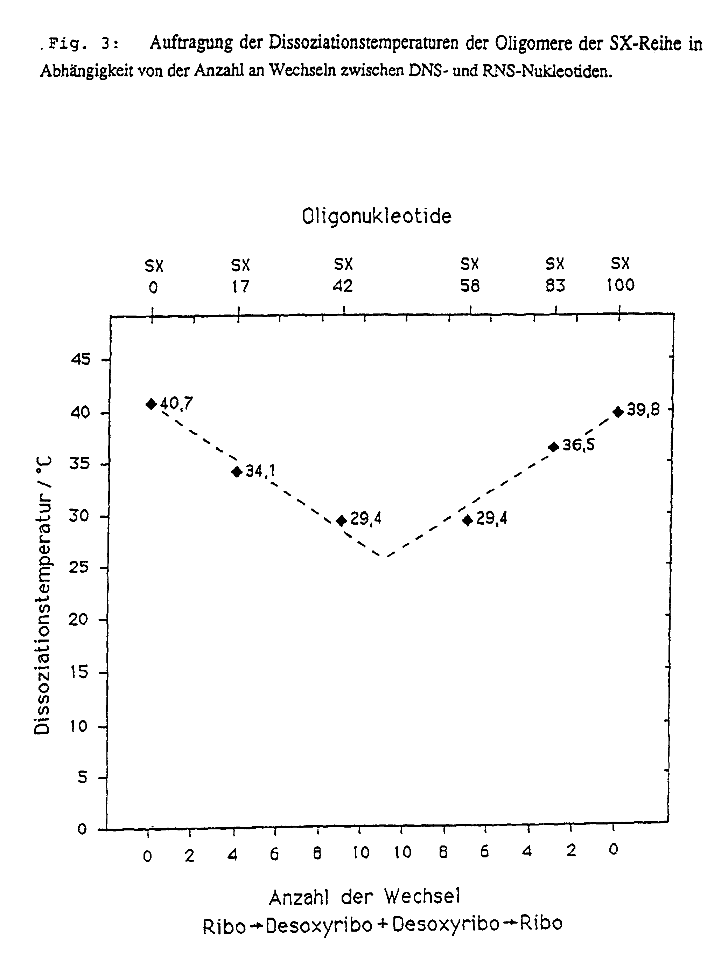

Durch obiges Experiment wurde u.a. auch die Dissoziationstemperatur der Oligomere bestimmt, d.h. die Temperatur bei der 50% des ursprünglich gebundenen Oligomers noch gebunden ist. Aus den Fig. 2 und 3 sieht man, daß mit zunehmender Zahl von Wechseln von DNA- zu RNA-Nukleotid bzw. von RNA- zu DNA-Nukleotid die Stabilität des Doppelstrangs linear abnimmt. Unter Wechseln ist die Zahl der Alternierungen zwischen DNA und RNA in einem Molekül zu verstehen. So finden in dem Fragment "AgCtTgAg" 7 Wechsel statt und in dem Fragment "AGCTTgag" nur 1 Wechsel.

Um die Bindungsstärke der Oligomere auf der Hybridisierungsmatrix ("Oligomer Chip") auf ein Niveau zu bringen, müssen die Dissoziationstemperaturen reiner DNA-Oligomere auf einem "Probe-Chip" mittels Hybridisierungen mit einem DNA-Testfragment bekannter Sequenz bestimmt werden. Da man von vornherein weiß, an welche Oligomere des "Probe-Chips" das Testfragment binden darf und an welche nicht (falsch Positive), bestimmt man von den Oligomeren an die das Fragment binden durfte und gebunden hat, die Dissoziationstemperatur. Diese Prozedur wiederholt man mit weiteren DNA-Testfragmenten bis von allen Oligomeren des Chips die Dissoziationstemperatur bestimmt worden ist. Dann nimmt man die ermittelte niedrigste Dissoziationstemperatur als diejenige an, auf die alle Oligomere eingestellt werden sollen, um alle Oligomere auf ein Niveau der Bindungsstärke zu bringen. Dies geschieht durch Einführung von RNA-Nukleotiden in die entsprechenden Oligomere bei der Synthese eines neuen Chips, d.h man führt entsprechende DNA/RNA-Wechsel ein und erniedrigt die Dissoziationstemperatur des Oligomers. Bevorzugt ist, daß die DNA/RNA-Hybridoligomere mindestens 2 Wechsel von DNA zu RNA bzw. von RNA zu DNA aufweisen, ganz bevorzugt sind mindestens 4 Wechsel. Auf dieser Grundlage erhält man einen Chip, der sich durch eine einheitliche Bindungsstärke aller Oligomere auszeichnet und für die Sequenzierung eines unbekannten DNA-Fragments geeignet ist. Im Umfang der Erfindung liegt auch, auf einem "Probe-Chip" RNA-Oligomere aufzubringen und auszutesten, um später DNA-Nukleotide einzubringen zur Herstellung der DNA-/RNA-Hybridoligomere.

Eine andere, jedoch nicht so genaue, Möglichkeit zur Voraussage der Bindungsstärke ist eine Berechnungsmethode auf Grundlage der Basensequenz. Diese Methode ist von Breslauer et al., (1986), Proc. Natl. Acad. Sci USA, 83, S. 3746-3750 beschrieben worden und kann unter Computereinsatz schnell einen Überblick liefern.

Die Erfindung wird nun weiter mit Bezug auf die Figuren beschrieben, von denen zeigen:
- Fig. 1: Prinzip der Oligomerhybridisierungs-Sequenzierung
- Fig. 2: Auftragung der Dissoziationstemperaturen der Oligomere der XS-Reihe in Abhängigkeit von der Anzahl an Wechseln zwischen DNA- und RNA-Molekülen
- Fig. 3: Auftragung der Dissoziationstemperaturen der Oligomere der SX-Reihe in Abhängigkeit von der Anzahl der Wechsel zwischen DNA- und RNA-Molekülen

## Patentansprüche

1. Verfahren zum Sequenzieren von DNA durch Oligomerhybridisierung, **dadurch gekennzeichnet, daß** als Hybridisierungsmatrix auf einem Träger aufgebrachte DNA-/RNA-Hybridoligomere und gegebenenfalls DNA-Oligomere und/oder RNA-Oligomere verwendet werden.

2. Verfahren gemäß Anspruch 1, **dadurch gekennzeichnet, daß** der Träger aus Glas, Polypropylen oder Silicium besteht.

3. Verfahren gemäß Anspruch 1 oder 2, **dadurch gekennzeichnet, daß** es sich bei der Hybridisierungsmatrix um eine Tetramer- bis Dodecamer-Matrix handelt.

4. Verfahren gemäß einem der Ansprüche 1 bis 3, **dadurch gekennzeichnet, daß** die Oligomere unter Verwendung photoaktivierbarer Bausteine und photolithographischer Techniken auf dem Träger synthetisiert worden sind.

5. Verfahren gemäß einem der Ansprüche 1 bis 4, **dadurch gekennzeichnet, daß** die Oligomere einzeln synthetisiert worden sind und dann mittels einem Linker auf die Trägeroberfläche aufgebracht worden sind.

6. Verfahren gemäß einem der Ansprüche 1 bis 5, **dadurch gekennzeichnet, daß** die DNA/RNA-Hybridoligomere mindestens vier Wechsel von DNA zur RNA bzw. RNA zu DNA aufweisen.

## Claims

1. A method of sequencing DNA by oligomer hybridization, **characterized by** using DNA/RNA hybrid oligomers applied onto a support as hybridization matrix and optionally DNA oligomers and/or RNA oligomers.

2. The method according to claim 1, **characterized in that** the support consists of glass, polypropylene or silicon.

3. The method according to claim 1 or 2, **characterized in that** the hybridization matrix is a tetramer to dodecamer matrix.

4. The method according to any of claims 1 to 3,
**characterized in that** the oligomers were synthesized on the support by using building blocks which can be photoactivated and photolithographic techniques.

5. The method according to any of claims 1 to 4,
**characterized in that** the oligomers were synthesized individually and were then applied on the support surface by means of a linker.

6. The method according to any of claims 1 to 5,
**characterized in that** the DNA/RNA hybrid oligomers have at least four cycles from DNA to RNA and/or RNA to DNA.

## Revendications

1. Procédé de séquençage d'ADN par hybridation d'oligomères, **caractérisé en ce qu'**on utilise comme matrice d'hybridation des oligomères hybrides ADN/ARN liés à un support et, le cas échéant, des oligomères d'ADN et/ou des oligomères d'ARN.

2. Procédé suivant la revendication 1, **caractérisé en ce que** le support est constitué de verre, polypropylène ou silicium.

3. Procédé suivant la revendication 1 ou 2,
**caractérisé en ce que** la matrice d'hybridation est une matrice de tétramère ou de dodécamère.

4. Procédé suivant l'une des revendications 1 à 3, **caractérisé en ce que** les oligomères ont été synthétisés sur le support avec utilisation de motifs photoactivables et selon des techniques photolithographiques.

5. Procédé suivant l'une des revendications 1 à 4, **caractérisé en ce que** les oligomères ont été synthétisés isolément puis attachés à la surface du support au moyen d'un linker.

6. Procédé suivant l'une des revendications 1 à 5, **caractérisé en ce que** les oligomères hybrides ADN/ARN présentent au moins quatre passages d'ADN à ARN ou d'ARN à ADN.
